Europäisches Patentamt

European Patent Office

Office européen des brevets

(11) Publication number: **0 058 325**
**B1**

(12) **EUROPEAN PATENT SPECIFICATION**

(45) Date of publication of patent specification: **27.03.85**

(21) Application number: **82100666.5**

(22) Date of filing: **30.01.82**

(51) Int. Cl.⁴: **A 61 M 1/14**

(54) **Flow-through chamber forming part of a blood duct.**

(30) Priority: **16.02.81 SE 8101025**

(43) Date of publication of application:
**25.08.82 Bulletin 82/34**

(45) Publication of the grant of the patent:
**27.03.85 Bulletin 85/13**

(84) Designated Contracting States:
**AT BE CH DE FR GB IT LI LU NL**

(56) References cited:
**FR-E- 71 790**
**GB-A-2 047 542**
**US-A-2 659 368**
**US-A-3 217 889**

(73) Proprietor: **GAMBRO DIALYSATOREN K.G.**
**Postfach 1323**
**D-7450 Hechingen (DE)**

(72) Inventor: **Heck, Wolfgang**
**Hechingerstrasse 29**
**D-745 Hechingen (DE)**

(74) Representative: **Boberg, Nils Gunnar Erik**
**Gambro AB Patent Department Box 10101**
**S-220 10 Lund (SE)**

Courier Press, Leamington Spa, England.

## Description

Technical Field

The present invention relates to a flow-through chamber, e.g. a drip chamber or an expansion chamber, intended to form part of a duct for blood or similarly sensitive fluids, comprising at least one inlet and at least one outlet and provided with means for the deflection of the fluid flow after the inlet.

In dialysis or similar extracorporeal blood treatments, for example, the patient is connected to the treatment machine with the help of a set of tubes which may comprise different types of chambers. In addition to the abovementioned example, the set of tubes may comprise different chambers for the connection of different measuring instruments, e.g. pressure gauges or thermometers.

The present invention relates to a chamber of this type, intended primarily to be used as an expansion chamber, but it will be clear to those versed in the art that it can also be used for other purposes.

Background Art

In chambers of the abovementioned type it is often considered desirable to deflect the fluid flow. Up to now this has been achieved normally with the help of additional loose parts, which were fixed in different ways to the chamber.

Reference is further made to FR—E—71790 in which a drip chamber is described provided with an internal local indentation inside a corresponding external indentation. No means are however provided inside the drip chamber for deflecting the fluid flow. Instead the liquid is dripping from the inlet at the top of the device entirely down to the actual liquid level.

Disclosure of the Invention

In the present invention the above mentioned loose parts have been eliminated in a simple manner in that the said means for the deflection of the fluid flow after the inlet is formed by the actual wall of the chamber whereby a part of the wall is indented to provide an internal local indentation to deflect the fluid flow and a corresponding external indentation.

The chamber in accordance with the invention is manufactured preferably by blow moulding with simultaneous formation of the said indented part. This can be achieved in a simple manner in that the blow mould is provided with an inwards projecting part corresponding to the desired indentation. Alternatively, the chamber can, of course, be manufactured, for example, by injection moulding or vacuum moulding. In this case it is made appropriately in the form of two halves which can be joined together.

The chamber in accordance with the invention is designed preferably so that it is of an elongated shape intended to be placed vertically with inlet and outlet arranged parallel at the bottom end in the form of nozzles designed as integral parts of the chamber. These nozzles are intended therefore to be connected to tubing included in the set of tubes. This is a suitable design if the chamber is to be used, for example, as an expansion chamber.

Such an expansion chamber may be required, for example, when a less expandable dialyser, e.g. a fibre dialyser, is used in the so-called "single-needle" technique. In this process blood is intermittently taken from the patient alternatingly with being intermittently returned to the patient in the same manner. An expansion chamber is needed here so that the pressure in the dialyser should not become too high. In many other types of dialyser no such expansion chamber is required, since the dialyser itself is capable of expanding and so prevent an increase in pressure.

In the use of such expansion chambers the subject of the invention is provided preferably with the inlet and the outlet arranged parallel at the bottom of the elongated chamber. It is appropriate for the outlet to be arranged at the lowest point of the chamber, whilst the inlet is arranged on a wall sloping obliquely towards this point. By this design in combination with the said means for deflection of the fluid flow a very gentle curvature of the fluid path from the inlet to the outlet can be obtained, which ensures careful treatment of the blood and at the same time prevents frothing.

The subject of the invention is of substantially the same shape also when it is used as a drip chamber. In this case, however, the inlet is arranged at the top end of the chamber and the outlet at its bottom end. The same means for deflection of the fluid flow may be arranged to reduce the height of drop of the fluid, if it does not reach as far as the said means. Here, too, a careful treatment of the blood or corresponding sensitive fluid which is to be conducted through the chamber is achieved. The indented part may be designed so that it causes the fluid flow to run quietly along the chamber wall instead of dropping freely down to the outlet. It may be gently curved, for example.

Brief Description of Drawings

In the following the invention will be described in greater detail with reference to the enclosed drawing which shows a preferred embodiment of the subject of the invention.

Figure 1 shows a longitudinal section through an expansion chamber.

Figure 2 shows a side elevation of the same chamber.

Figure 3 shows in the same manner an end elevation of the chamber and

Figure 4 shows a section along the line IV—IV in figure 1.

Best Mode of Carrying Out the Invention

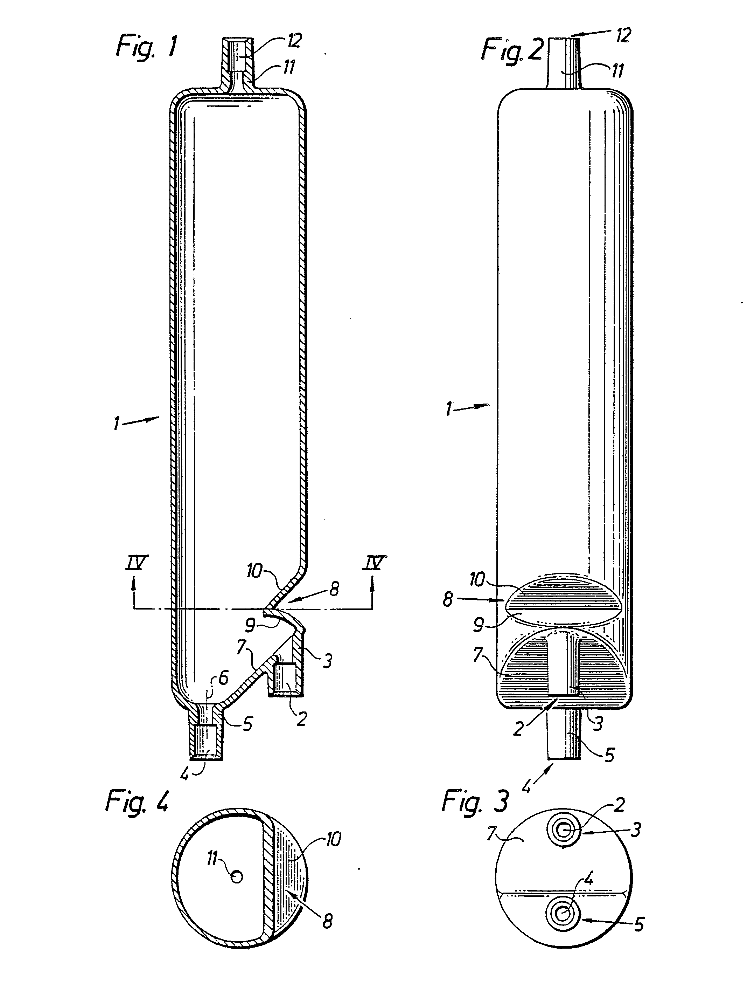

Fig. 1 thus shows an expansion chamber designed in accordance with the invention. However, it will be clear to those versed in the art, that this chamber, with small modifications, can easily be altered to become, for example, a drip chamber or some other chamber included in a set of tubes of the aforementioned type.

The expansion chamber as a whole is designated 1 and it is provided with an inlet 2 arranged in an inlet nozzle 3 and an outlet 4 arranged in an outlet nozzle 5. It can be said that the outlet is arranged so that it issues from the lowest point 6 of the chamber. In the same manner it can be said that the inlet 2 is arranged to open out into a wall 7 sloping obliquely towards this point 6.

The indentation characterizing the invention, which thus constitutes the special means for the deflection of the fluid flow, is designated as a whole by numeral 8 and consists of a lower wall portion 9 and an upper wall portion 10. The lower wall portion 9, seen from the inside, is of a concave design so that the fluid flow should be deflected gently in the desired direction towards the outlet 4. The upper wall portion 10 may, in principle, be of any shape.

In the example shown the expansion chamber is designed with a third nozzle 11 with outlet 12 which may be connected for example to a pressure gauge for measuring the pressure in the chamber.

Naturally, the invention is not limited exclusively to the embodiment of the subject of the invention described above, but can be varied within the scope of the following claims. As mentioned above, it will be clear to those versed in the art that the chamber can readily be modified for different uses. If the chamber is to be used, for example, in upside down condition in relation to the drawing, the indentation 8 will be designed appropriately more gently curved, so as to force the fluid flow to follow the wall portions 9 and 10.

## Claims

1. A flow-through chamber, e.g. a drip chamber or an expansion chamber (1), intended to form part of a duct for blood or similarly sensitive fluids, comprising a least one inlet (2) and at least one outlet (4) and provided with means (8) for the deflection of the fluid flow after the inlet (2), characterized in that the said means (8) is formed by the actual wall of the chamber (1), whereby a part (8) of the wall is indented to provide an internal local indentation to deflect the fluid flow and a corresponding external indentation.

2. A chamber in accordance with claim 1, characterized in that the flow-through chamber is manufactured by blow molding with simultaneous formation of the said indented part (8).

3. A chamber in accordance with any one of claims 1 or 2, characterized in that the flow-through chamber is of an elongated shape intended to be placed vertically with inlet (2) and outlet (4) arranged parallel at the bottom end (6) in the form of nozzles (3 and 5, respectively) designed as integral parts of the chamber.

4. A chamber in accordance with claim 3, characterized in that the outlet (4) is arranged at the lowest point (6) of the chamber whilst the inlet (2) is arranged on a wall (7) sloping obliquely towards this point (6).

5. A chamber in accordance with any one of claims 1 or 2, characterized in that the flow-through chamber is of an elongated shape intended to be placed vertically with an inlet (2) arranged at the upper end and an outlet (12) arranged at the lower end the said means (8) for deflecting of the fluid flow being arranged so as to reduce the height of drop of the fluid, if the same does not reach as far as the said means (8).

## Revendications

1. Chambre de passage, telle qu'une chambre de goutte à goutte ou une chambre d'expansion (1), destinée à faire partie d'un conduit pour le sang ou des fluides de sensibilité analogue, comprenant au moins un orifice d'entrée (2) et au moins un orifice de sortie (4) et comportant des moyens (8) pour la déviation du flux de fluide au-delà de l'orifice d'entrée (2), caractérisée en ce que les moyens précités (8) sont constitués par la paroi même de la chambre (1), une partie (8) de ladite paroi étant indentée pour définir une indentation locale interne afin de dévier le flux de fluide et une indentation externe correspondante.

2. Chambre suivant la revendication 1, caractérisée en ce que la chambre de passage est fabriquée par moulage par soufflage, avec formation simultanée de la partie indentée précitée (8).

3. Chambre suivant l'une des revendications 1 ou 2, caractérisée en ce que la chambre de passage est de forme allongée, destinée à être disposée verticalement, avec l'orifice d'entrée (2) et l'orifice de sortie (4) disposés en parallèle à l'extrémité inférieure (6) de la chambre, sous forme de buses (3 et 5 respectivement) formées d'un seul tenant avec la chambre.

4. Chambre suivant la revendication 3, caractérisée en ce que l'orifice de sortie (4) est disposé au point le plus bas (6) de la chambre, tandis que l'orifice d'entrée (2) est disposé sur une paroi (7) s'inclinant obliquement en direction de ce point (6).

5. Chambre suivant l'une des revendications 1 ou 2, caractérisée en ce que la chambre de passage est de forme allongée destinée à être disposée verticalement, avec un orifice d'entrée (2) disposé à l'extrémité supérieure de la chambre et un orifice de sortie (12) disposé à l'extrémité inférieure de la chambre, les moyens précités (8) de déviation du flux de fluide étant agencés de manière à réduire la hauteur de chute du fluide si celui-ci n'arrive pas jusqu'auxdits moyens.

## Patentansprüche

1. Durchflußkammer, z.B. eine Tropfkammer oder eines Dehnungskammer (1), für die Bildung eine Teiles einer Blutleitung oder einer Leitung für ähnlich empfindliche Fließmittel, mit mindestens einem Einlaß (2) und mindestens einem Auslaß (4) und mit einer Einrichtung (8) für die Umlenkung des Fließmittelstromes nach dem Einlaß (2), dadurch gekennzeichnet, daß die Einrichtung (8) durch die eigentliche Wand der Kammer (1) gebildet ist, wobei ein Teil (8) der Wand vertieft ist, um für die Umlenkung des Fließmittelstromes eine innere örtliche Vertiefung und eine entsprechende äußere Vertiefung vorzusehen.

2. Kammer nach Anspruch 1, dadurch gekennzeichnet, daß die Durchflußkammer durch das Blasformverfahren unter gleichzeitiger Bildung des vertieften Teiles (8) hergestellt wird.

3. Kammer nach einem der Ansprüche 1 oder 2, dadurch gekennzeichnet, daß die Durchflußkammer eine längliche Gestalt hat, um vertikal angeordnet zu werden, wobei der Einlaß (2) und der Auslaß (4) am Boden (6) parallel angeordnet und in der Form von Düsen (3 bzw. 5) als einstückige Teile der Kammer ausgestaltet sind.

4. Kammer nach Anspruch 3, dadurch gekennzeichnet, daß der Auslaß (4) an der untersten Stelle (6) der Kammer angeordnet ist, während der Einlaß (2) auf einer sich schräg zu dieser Stelle (6) hin neigenden Wand (7) angeordnet ist.

5. Kammer nach einem der Ansprüche 1 oder 2, dadurch gekennzeichnet, daß die Durchflußkammer längliche Gestalt hat, so daß sie vertikal angeordnet wird, wobei ein Einlaß (2) am oberen Ende und ein Auslaß (12) am unteren Ende angeordnet sind, und die Einrichtung (8) zum Umlenken des Fließmittelstromes so angeordnet ist, daß sie die Tropfhöhe des Fließmittels verringert, wenn dieselbe nicht soweit reicht wie die Einrichtung (8).

Fig. 1

Fig. 2

Fig. 4

Fig. 3